# EUROPEAN PATENT SPECIFICATION

(11) **EP 1 799 838 B1**
(45) Date of publication and mention of the grant of the patent: **15.12.2010**
(21) Application number: 05796122.9
(22) Date of filing: 07.10.2005
(51) Int. Cl.: C12P 21/06, A23J 3/34, C07K 14/575, C07K 14/72

(54) **METHODS OF PRODUCING BIOACTIVE COMPOSITIONS**
VERFAHREN ZUR HERSTELLUNG VON BIOAKTIVEN ZUSAMMENSETZUNGEN
PROCEDES DE PREPARATION DE COMPOSITIONS BIOACTIVES

(30) Priority: 07.10.2004 US 617021 P
(43) Date of publication of application: 27.06.2007
(73) Proprietor: Nestec S.A., 1800 Vevey (CH)
(72) Inventor: GRIGOROV, Martin, CH-1066 Epalinges (CH); VAN BLADEREN, Peter, CH-1071 Chexbres (CH); ZACHARIAE, Ulrich, 37081 Goettingen (DE); KOCHHAR, Sunil, CH-1073 Savigny (CH); FAY, Laurent-Bernard, F-74500 Evian (FR); CORTHESY-THEULAZ, Irène, CH-1066 Epalinges (CH); JUILLERAT, Marcel-Alexandre, CH-1000 Lausanne 26 (CH); AFFOLTER, Michael, CH-1073 Savigny (CH)
(74) Representative: Marquardt, Ulf
(86) International application number: PCT/EP2005/010837
(87) International publication number: WO 2006/037655

(56) References cited:
- WO-A-03/026591
- WO-A-03/082019
- WO-A-03/082019
- WO-A-2004/057976
- WO-A-2004/057976
- US-A- 3 896 103
- LOPONEN, J. ET AL: "Angiotensin converting enzyme inhibitory peptides in Finnish cereals: a database survey" AGRICULTURAL AND FOOD SCIENCE, vol. 13, no. 1-2, January 2004 (2004-01), pages 39-45, XP008067115
- DZIUBA, J. ET AL.: "Database of biologically active peptide sequences" NAHRUNG, vol. 43, no. 3, 1999, pages 190-195, XP008067114 WEINHEIM, DE
- FUJITA HIROYUKI ET AL: "Isolation and characterization of ovokinin, a bradykinin B-1 agonist peptide derived from ovalbumin" PEPTIDES, ELSEVIER, AMSTERDAM, US, vol. 16, no. 5, 1995, pages 785-790, XP002291360 ISSN: 0196-9781
- YOSHIKAWA M ET AL: "BIOACTIVE PEPTIDES DERIVED FROM FOOD PROTEINS PREVENTING LIFESTYLE-RELATED DISEASES" BIOFACTORS, OXFORD UNIVERSITY PRESS, OXFORD, GB, vol. 12, no. 1-4, 2000, pages 143-146, XP001007186 ISSN: 0951-6433
- SHI LEI ET AL: "BioPD: a web-based information center for bioactive peptides" REGULATORY PEPTIDES, vol. 120, no. 1-3, 15 August 2004 (2004-08-15), pages 1-3, XP002392268 ISSN: 0167-0115
- DATABASE UniProt 1 March 2001 (2001-03-01), SATO, S ET AL.: "Arabidopsis Amylogenin" XP002463879 retrieved from EBI accession no. Q9FFD2_ARATH Database accession no. Q9FFD2
- DATABASE UniProt 1 May 1999 (1999-05-01), DE PATER, S. ET AL.: "Oryza Amylogenin" XP002463880 retrieved from EBI accession no. Q9ZR36_ORYSA Database accession no. Q9ZR36
- DATABASE UniProt 1 November 1998 (1998-11-01), BLIGHT, F.H. ET AL.: "Oryza RPG2" XP002463881 retrieved from EBI accession no. O82706_ORYSA Database accession no. O82706
- DATABASE UniProt 1 November 1996 (1996-11-01), KALINSKI, A. ET AL.: "Soybean BiP" XP002463882 retrieved from EBI accession no. Q39803_SOYBN Database accession no. Q39803
- LOPONEN, J. ET AL: "Angiotensin converting enzyme inhibitory peptides in Finnish cereals: a database survey" AGRICULTURAL AND FOOD SCIENCE, vol. 13, no. 1-2, January 2004 (2004-01), pages 39-45, XP008067115
- DZIUBA, J. ET AL.: "Database of biologically active peptide sequences" NAHRUNG, vol. 43, no. 3, 1999, pages 190-195, XP008067114 WEINHEIM, DE
- FUJITA HIROYUKI ET AL: "Isolation and characterization of ovokinin, a bradykinin B-1 agonist peptide derived from ovalbumin" PEPTIDES, ELSEVIER, AMSTERDAM, US, vol. 16, no. 5, 1995, pages 785-790, XP002291360 ISSN: 0196-9781
- YOSHIKAWA M ET AL: "BIOACTIVE PEPTIDES DERIVED FROM FOOD PROTEINS PREVENTING LIFESTYLE-RELATED DISEASES" BIOFACTORS, OXFORD UNIVERSITY PRESS, OXFORD, GB, vol. 12, no. 1-4, 2000, pages 143-146, XP001007186 ISSN: 0951-6433
- KONDO H ET AL: "Cloning and sequence analysis of the genomic DNA fragment encoding oryzacystatin" GENE, ELSEVIER, AMSTERDAM, NL, vol. 81, 1989, pages 259-265, XP000652087 ISSN: 0378-1119
- SHI LEI ET AL: "BioPD: a web-based information center for bioactive peptides" REGULATORY PEPTIDES, vol. 120, no. 1-3, 15 August 2004 (2004-08-15), pages 1-3, XP002392268 ISSN: 0167-0115
- IZQUIERDO-PULIDO ET AL: JOURNAL OF AGRICULTURAL AND FOD CHEMISTRY, vol. 42, no. 3, 1994, pages 616-622,

## Description

### BACKGROUND OF THE INVENTION

The genomics-proteomics revolution of the 1990's has led to the identification and design of numerous small molecule drugs and potential drugs. In many cases these compounds do not occur in nature and many of them are toxic, antigenic, or have unsuitable pharmacokinetic properties.

Many bioactive molecules occur naturally in food stuffs as part of larger precursor molecules. Because they are components of foods that are commonly eaten, such agents have the potential to be less toxic to humans and animals. If methods could be found to release these compounds from their precursor food sources these molecules could be utilized to enrich foods or could be isolated and used as nutritional or therapeutic agents.

For example, PYY is a high affinity positive agonist of Y2 G-protein-coupled receptors (GPCR) and represents a relatively new class of therapeutic treatment for obesity, among other diseases. It is a natural hormone produced by specialized endocrine L-cells in the gut in proportion to the calorie content of a meal. PYY operates by reducing appetite and food intake by modulating appetite circuits in the hypothalamus. The agent has been shown to reduce caloric intake by 30% two hours after subjects, either obese or nonobese, received a 90-minute intravenous infusion. These subjects also experienced a significant decrease in their cumulative 24-hour caloric intake. In other studies, obese individuals have been observed to have lower levels of circulating PYY.

### SUMMARY OF THE INVENTION

The present invention provides a process in accordance with claim 1, a method in accordance with claim 6, a method in accordance with claim 10, and a food product in accordance with claim 15. The procedure first involves identifying the functional biomolecules consisting of a peptide defined by the amino acid sequence of SEQ-ID No. 8 or 9 to be generated.

Precursor molecules containing this consensus motif, with all of the chemical and structural attributes required for activity, are then identified by electronically searching genomic databases of a variety of precursor food sources. Once a potential precursor molecule and food source is identified, another database search is carried out to identify organisms or enzymes that can be used to release from the precursor molecule a smaller compound containing all the attributes identified as necessary for function. The functional molecule can then be generated by treating the precursor with the processing agent(s) and releasing the functional biomolecule from its precursor. Functional molecules include bioactive molecules or biomaterials wherein the functions are bioactivity and self-organization and assembly, respectively. Precursor food sources include food stuffs or food grade proteins and their mixtures.

This approach provides a novel method for processing genome information in the design of combinations of raw nutritional sources and organisms which can liberate, *in situ,* active small molecules to enhance the nutritional and health-enhancing effect of the ingested functional food products.

Dziuba et al, Nahrung 43, 1999, 190-195 describes that is possible to predict biological activity of protein fragments using sequence alignments between proteins and biologically active peptides from a database. These sequence alignments can give information about localization of biologically active fragments in a protein chain, but not about possibilities of enzymic release of such fragments.

US-A-3896103 discloses a peptide having the amino acid sequence PWMDF.

Kondo et al., Gene, Elsevier, Amsterdam, NL, vol 81, 1989, pages 259-265 discloses oryzacystatin.

Izquierdo-Pulido et al. describe in J. Agric. Food. Chem., 42, 1994, page 616-622, the presence of oryzacystatin and other protease inhibitors in 11 major cultivars of rice that can be used for inhibiting gel weakening in cooked surimi.

To this end the invention provides a method for generating a bioactive molecule consisting of a peptide defined by the amino acid sequence of SEQ-ID No. 8 or 9 from a precursor food or protein source. The method includes the steps of identifying a precursor food source that contains a precursor molecule comprising the functional biomolecule identifying an agent that can be used to release from the precursor molecule said functional biomolecule, and treating the precursor food source with the processing agent to thereby release from the precursor molecule said functional biomolecule.

In another aspect of the method the precursor food source can be derived from a plant such as rice, soy, maize, potato, coffee, milk, meat, and the like.

In another aspect, the method involves preparing a biomolecule using an agent that is a cell and the cell can be a lactobacteria, including *Lactobacillus johnsonii* (La1) or bifidobacteria, such as *Bifidobacterium longum* (B129) for example.

In another aspect, the method involves preparing a biomolecule using an agent that is an enzyme.

In another aspect, the method involves preparing a biomolecule using an agent that is an enzyme such as a protease. .

In another aspect of the invention the biomolecule binds to a receptor.

Certain embodiments of the invention are directed to a bioactive agent consisting of a peptide defined by the amino acid sequence of SEQ-ID No. 8 or 9.

In certain embodiments of the invention the functional agent is isolated from a food stuff and the food stuff is rice.

In certain embodiments of the invention the food stuff is *Oryza sativa.*

The bioactive agent of the invention is a peptide that is defined by the sequence PWMDF (SEQ ID No. 8), or VWEKPWMDF (SEQ ID No. 9) all derived from *Oryza sativa* oryzacystatin.

In certain embodiments of the invention the functional agent is generated by releasing it from a precursor molecule.

In certain embodiments of the invention the functional agent is generated by releasing it from a precursor molecule wherein the precursor molecule is in rice.

Additional features and advantages of the present invention are described in, and will be apparent from the following Detailed Description of the Invention.

### DETAILED DESCRIPTION OF THE INVENTION

For purposes of this specification the terms bioactive molecule and biomaterial are both considered to be functional biomolecules. The terms can be used interchangeably so long as their unique functions are kept in mind. Thus, methods and compositions described in this specification with respect to bioactive molecules are equally applicable to biomaterials.

Biomaterials are generally considered to be molecules that are capable of self-organization and assembly. Thus, biomaterials can be peptides that can form filaments and fibrils, hydrogels, surfactants and peptide hybrids when released from their precursor molecules.

The present invention provides methods for producing functional foods that are enriched in bioactive peptides consisting of a peptide defined by the amino acid sequence of SEQ-ID No. 8 or 9, using natural food sources and food grade proteins. In addition, the present invention relates to the foods so designed.

In an embodiment, the present invention is directed to methods for developing natural bioprocessing procedures for producing enriched foods upon treatment with naturally occurring biological molecules, processing activities present in select organisms or extracts of such organisms or other natural processing agents. The invention also covers the various food compositions that can be prepared by the procedures, in addition to the bioactive molecules and methods for their production.

In the case of a bioactive molecule, the method generally involves identifying a bioactive molecule consisting of a peptide defined by the amino acid sequence of SEQ-ID No. 8 or 9 for production, identifying a precursor food source that contains a precursor molecule with those attributes, identifying an agent or agents that can be used to release from the precursor molecule a compound comprising the attributes necessary for bioactivity. Once a suitable processing method is identified, the method can be carried out by treating the precursor food source with the processing agent(s) to cause the release of a molecule that contains the attributes necessary for bioactivity.

The method also desirably includes steps for assessing the abundance of the potential precursor molecules. In addition, where the functional agent is derived from a precursor protein, the method also desirably includes an assessment of the stability of the potential precursor proteins towards peptidases and other enzymes or treatments that can be used to release it.

The functional agent is a bioactive molecule consisting of a peptide defined by the amino acid sequence of SEQ-ID No. 8 or 9. The functional agents can be excised from food stuffs, including food grade proteins, or other agricultural sources by the present methods. Generally, the desired bioactive molecules will specifically bind to a target receptor and activate or deactivate the receptor at concentrations that are obtainable from food processing. In certain embodiments where concentrations of the functional agent are lower than desired, chosen functional agents can be concentrated in the food source. Alternatively, extracts of the processed food stuffhaving elevated concentrations of functional agents can be prepared. Purification methods can also be used to purify agents partially or to substantially pure form. Methods for preparing concentrates, extracts, and for purification of functional agents necessarily vary depending on the nature of the target functional agent but these methods are well known in the art and can be easily carried out by one of skill in the art.

Precursor molecules can also be concentrated or purified and treated to release the bioactive molecules which can then be added back to food stuffs, as desired.

A precursor food source that contains a molecule consisting of a peptide defined by the amino acid sequence of SEQ-ID No. 8 or 9 can then be identified. Such food sources can be identified by searching for the occurrence of the consensus motif at both sequence and structural levels in large collections of genomic information including for example, ENSEMBL®, Nestlé genomic databases, and public genomic sequencing projects on food raw materials such as rice, soy, maize, potato, coffee and bovine milk. Precursor molecules found within these food sources can be proteins or peptides and their derivatives. Preferably since the agent is a peptide, such a search is performed using BLAST by searching for short nearly exact matches and/or by EMBOSS pattern matching module "patmatdb" by searching for short nearly exact matches. Where three dimensional structural motifs are required these structures can be found using structure prediction software, as is known in the art.

It is envisioned that a number of potential precursor molecules may be identified by this method. In such cases, it can be desirable to determine the abundance of the precursor molecules in order to identify precursors that will yield suitable amounts of the desired agent.

Alternatively, food grade proteins containing the consensus motif can be obtained and used in the preparation of the biomolecule.

A processing agent or agents can then be identified that can be used to release a compound from the precursor molecule such that the released compound contains all of the attributes necessary for bioactivity. The selected agents will, of course, depend on the nature of the functional agent to be released and the characteristics of the matrix in which it is found. Potential agents include microorganisms, extracts of microorganisms, proteolytic, glycolytic, nucleolytic, and lipolytic enzymes, oxidases, glycosidases, and chemical agents. In some instances these activities can be found in the genome repertoire of cells, such as probiotic bacteria.

Where proteases are required, the automated service offered by ExPASy website termed PeptideCutter, the module DIGEST from the bioinformatics software package EMBOSS, and/or the MEROPS protease database can be used to identify suitable enzyme activities. As is known, the PeptideCutter knowledge-based algorithm can be used to identify cleavage sites produced by a panel of more than 20 different proteases on protein sequences. (Keil, B., Specificity of Proteolysis, Springer-Verlag Berlin-Heidelberg-New York, 1992)

Certain methods utilize microorganisms to release the functional agent from its matrix. To identify microorganisms containing the desired activities, such as protease activities, the sequences of the enzymes thought to be useful can be compared to known bacterial genomes to identify similar protease sequences in those genomes. For example, the *Bifidobacterium longum* (B129) genome contains 74 protein sequences annotated as proteases or peptidases. One of them is highly similar to an Arg-C proteinase for which cleavage outcomes are computationally predictable by the PeptideCutter model. Thus, where an Arg-C proteinase activity is identified as a potential activity for use in releasing the bioactive agent from its source, the bacterium *Bifidobacterium longum* (B129), which contains that sequence, could be used The *Bifidobacterium longum* (B129) genome also has a sequence highly similar to sedolisin. It can be used in situations where both activities would be useful in releasing the bioactive agent from its precursor food source. This method is equally applicable with other enzyme activities.

Alternatively, the activities of probiotic bacterial strains can be identified and utilized without specific knowledge of the nature of the enzymes involved. At least with respect to proteases both intracellular and extracellular activity profiles have been reported. In a similar manner glycolytic, nucleolytic, and lipolytic activities can also be screened and the entire NCC bacterial collection evaluated to select the most promising strains.

Once both a precursor molecule is identified within a food source and processing agents are identified for releasing the functional agent, the method can be carried out by treating the food source with the processing agent(s) to release from the precursor molecules, molecules that contain the consensus motif. Preferably, the food source is treated with agents that are sufficient to release the functional agent in a single fermentation or treatment. However, when multiple processing agents are required and they are incompatible or require distinct environments for use, multiple processing steps can be undertaken.

The functional agent can be derived from a protein or peptide sequence. In such cases, precursor molecules can be identified for example by retrieving all known plant expressed sequence tags and proteins that contain the sequence of the bioactive agent. Variable or noncritical amino acids and critical amino acids in the sequence can be identified by analysis of published structure-activity relationships and by aligning the sequences of all known similar sequences. With this consensus motif a database containing all known plant proteins can be searched to identify the consensus motif within larger precursor proteins. A computational assessment of the tertiary structure can also be used to identify internal sequences in the identified precursors that will adopt a structure that is required for activity of the bioactive agent. This can be done using standard methods well known in the art. For example, where peptide sequences closely resemble known three dimensional structures, homology-modeling can be used. In cases where peptide sequences are more distant to known structures, fold-recognition protocols can be used. Where three-dimensional structure information of the receptor for the bioactive agent is available, the identified sequences can be analyzed by a variety of known modeling methods to determine whether their predicted structures are likely to bind to the receptor. The list of precursor proteins is then evaluated. An analysis can then be done by known methods, such as microarrays for genes differentially expressed in seeds and crops, to determine if the precursor molecule is sufficiently abundant to enable the preparation of biologically relevant amounts of the functional agent from the precursor. In addition, the sensitivity of the precursor molecule to proteases can be determined in order to evaluate the potential for releasing the target bioactive agent. Peptide synthesis of identified peptide sequences can be carried out and the peptides can then be tested for activity.

In one exemplary embodiment the generation of analogues to the bioactive peptide PYY3-36 can be selected. This compound is a ligand of the GPCR peptide hormone receptor Y, existing as subtypes Y1, Y2, Y4, and Y5. The receptor is involved in the regulation of satiety, the feeling of hunger. The PYY3-36 peptide activates its target receptors Y1 and Y2 at concentrations of about 0.5 nM. The method would also be useful in the identification and generation of other regulatory peptides against diseases such as diabetes and obesity, such as cholecystokinin (CCK), human growth hormone (HGH), and melanocortin, for example.

By way of example and not limitation, examples of the present invention can now be set forth.

### EXAMPLE 1

This example demonstrates the preparation of a bioactive agent having a positive agonist activity similar to PYY3-36. PYY3-36 is a ligand of the GPCR peptide hormone receptor Y, existing as subtypes Y1, Y2, Y4, and Y5. The receptor is involved in the regulation of satiety, the feeling of hunger, and blood pressure. It activates its target receptors Y1 and Y2 at concentrations of about 0.5 nM.

### Definition of the simplest bioactive molecular framework:

An extensive literature search was conducted and was used to determine that only a small sequence in the C-terminal part PYY3-36 peptide is essential for appetite regulation and for binding to its cognate receptor. Variations around this sequence segment have been published. Several published sequences are gathered and shown below in Table 1. The amino acids in bold in Table 1 are highly conserved amino acids that constitute the simplest bioactive framework for PYY3-36.

NMR structure determination techniques have shown that the PYY3-36 peptide adopts a very particular three-dimensional shape known as the PP-turn type or fold. The N-terminal region is unstructured, while the C-terminal region forms a characteristic α-helical structure. Using this information, peptides having similar sequences and tertiary structures were identified to find potential homologues to the natural PYY3-36 ligand peptide.

The search was performed using EMBOSS "patmatdb" with the following pattern reproducing at best the chemical nature of the C-terminal fragment of the PYY3-36 - XXX[RK]X[YFW]XXXX[TS][RK]X[RK][YFW]. Interestingly, tens of matching fragments occurring in various plant genomes were identified, several examples of which are shown in Table 1. The existence of the required α-helix structures of the target fragments was confirmed using peptide structure prediction software.

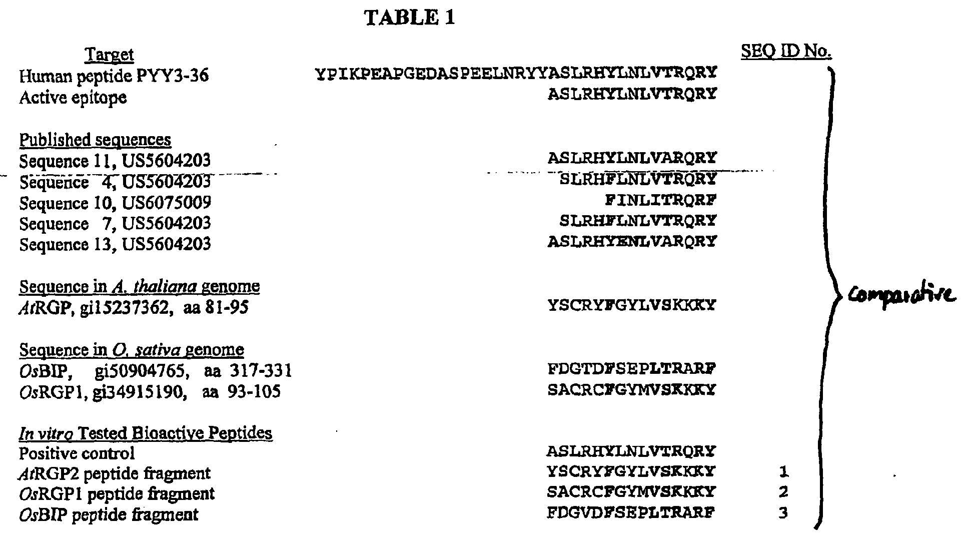

### Identified Hits for PYY22-36 Analogues:

Table 1 also identifies three potential bioactive peptides resulting from a search of known ESTs using the EMBOSS program. These sequences include two peptides from the protein *Amylogenin,* one from *Arabidopsis thaliana* (*At*RGP2), the other from *Oryza sativa* (*Os*RGP1). The other potential peptide sequence was from a binding protein found in *Oryza sativa* (*Os*BiP).

*Amylogenin* is thought to be responsible for starch biosynthesis in plants and is also known as reversibly glycosylated protein (RGP). Analysis of *Arabidopsis thaliana* seed microarrays and microbiological data suggest that it is an abundant protein in seeds and roots, and that it is almost exclusively localized in plant Golgi membranes. BiP or binding protein is responsible for enhancing crop tolerance to environmental stress. Microbiological data suggest that BiP synthesis is coordinated with the onset of active storage protein in crops.

The peptides derived from the proteins *At*RGP2, *Os*RGP1 and OsBiP were synthesized in 5 mg quantities at and purified to a purity of above about 90%. The peptides were tested in a competitive binding assay against PYY²²⁻³⁶ for binding to the GPCR receptor.

### Identifying target bioactive molecular frameworks. Search for adequate proteolytic enzymes in the genome repertoire of Nestle probiotic bacteria:

The automated free service offered by ExPASy website termed "PeptideCutter" and the module Digest from the bioinformatics software package EMBOSS was used to identify proteases that could release the PYY³⁻³⁶ analogue. Arg-C proteinase and sedolisin were identified as having activities that could release the bioactive agent from its native matrix. The sequences of the proteases used by PeptideCutter, MEROPS and Digest were compared to several bacterial genomes to check for the occurrence of highly similar sequences. For instance, in the *Bifidobacterium longum* genome 74 protein sequences are annotated as proteases or peptidases. Only one of them was found similar to the Arg-C proteinase for which cleavage outcomes are computationally predictable by the PeptideCutter model. Arg-C proteinase activity alone was not capable of producing the required active peptide. However, in the *Bifidobacterium longum* genome a sequence similar to sedolisin was identified. The combined activity of these two proteases in *Bifidobacterium longum* was shown to be capable of processing the *Oryza sativa* precursor protein into the desired PYY homolog.

### In vitro/In vivo tests for bioactivity:

*In-vitro* receptor-based screening of receptor subtypes Y1, Y2, and Y3 was performed according to protocols known in the art. (Munoz et al., Mol. Cell. Endocrinol. 107, 77 (1995), Fuhlendorf et al., PNAS 87, 182 (1990)) Screening of OsBiP, ZmRGP1 and AtRGP1 was conducted on Y2-GPCR as described above using known methods and activity was measured as the percentage of inhibition of binding of the endogenous ligand PYY²²⁻³⁶ sequence. The PYY²²⁻³⁶ sequence was used as a positive control. Of the peptides tested, the one derived from *At*RGP1 protein was able to inhibit binding of PYY²²⁻³⁶ by 20-40% when 10 µM concentration of both the PYY²²⁻³⁶ and *O*sBiP peptide were used in the binding assay. It is expected that the close analogue of the peptide derived from the *Arabidopsis Thaliana* protein *At*RGP1 found in the rice protein *O*sRGP2, differing by only one chemically equivalent amino acid, should have similar activity. Using this technique OsBIP protein was also shown to bind the Y2 receptor the Y2 receptor. *In vivo* tissue-based screening on cultures of rat colon cells can be carried out according to known protocols, such as described in Dumont et al., Eur. J. Pharmacol. 238, 37 (1993).

### EXAMPLE 2

This example demonstrates the preparation of a bioactive agent that inhibits the effect of peptide CCK-4 activator on the CCK-B subtype receptor. CCK-8 and CCK-4 peptides are ligands of the GPCR peptide hormone receptor CCK, which exists in at least two subtypes, A and B. CCK receptor subtype A is thought to be expressed in the gut and to be involved in the regulation of satiety, and the feeling of hunger. CCK receptor subtype B, also known as gastrin receptor, is thought to be involved in the secretion of gastric acid, and in the development of pathological conditions, such as of gastric ulcer and cancer. Antagonists of the CCK-B receptor can be used to treat these conditions.

### Definition of the simplest bioactive molecular framework:

A common strategy in the design of antagonists is to mimic a positive agonist and to introduce molecular structure changes that enhance binding to the cognate receptor such that the antagonist occupies the binding site thereby preventing agonist binding. An extensive literature search and sequence comparison was used to determine that only a small sequence in the C-terminal part of human CCK peptides is essential for receptor binding. A summary of this information is provided in Table 2. The amino acids in bold in Table 2 are highly conserved amino acids that constitute the simplest bioactive framework for CCK peptides.

The search was performed using EMBOSS "patmatdb" with the following pattern representing the chemical nature of the C-terminal fragment of CCK-peptides, - P[YFW]X[DE][YFW]. Tens of matching fragments occurring in various plant genomes were identified, several examples of which are shown in Table 2.

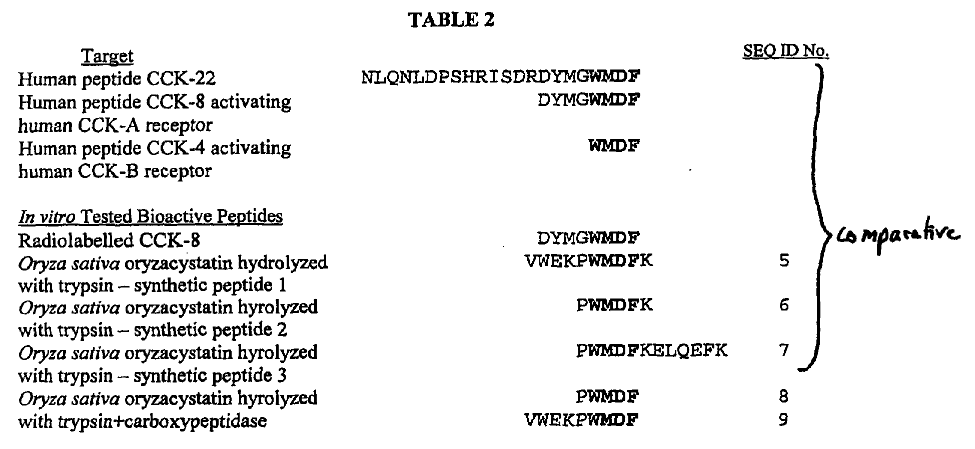

### Identified Hits:

Table 2 also identifies two potential bioactive peptides from a search of known ESTs using the EMBOSS program. These two sequences include two fragment peptides from the protein oryzacystatin, from the organism *Oryza sativa.*

Oryzacystatin is a cystein proteinase inhibitor protein. Analysis of literature demonstrated that this protein is produced in high quantities in rice crops, that could reach 1mg of protein per kilogram of crops. The peptides VWEKPWMDFK, PWMDFK and PWMDFKELQEFK were synthesized in 5 mg quantities and purified to a purity of above about 90%. The peptides were tested in a competitive binding assay against CCK-8 for binding to the CCK-B GPCR receptor.

### Identifying target bioactive molecular frameworks. Search for adequate proteolytic enzymes:

Commercial proteolytic enzymes such as trypsin or carboxypeptidase have been used to generate the active fragments from *Oryza sativa* rice.

### In vitro tests for bioactivity:

*In-vitro* receptor-based screening of receptor subtypes CCK-A and CCK-B was performed according to protocols known in the art. The binding activity of peptides VWEKPWMDFK, PWMDFK and PWMDFKELQEFK on CCK-A and CCK-B GPCR was measured as the percentage of inhibition of binding of the endogenous ligand CCK-8 sequence. The CCK-8 sequence was used as a positive control in this assay. Results showed that peptides VWEKPWMDFK, PWMDFK and PWMDFKELQEFK inhibited binding of the ligand CCK-8 to CCK-B receptor by 27%, 16%, and 5%, respectively at 10 µM concentration. None of the peptides tested activated CCK-A receptor at concentrations as high as 10 µM, probably due to the lack of sulfonation, a chemical modification required for agonizing the CCK-A receptor.

Hydrolysates were generated from the recombinantly expressed and purified oryzacystatin protein which was isolated from *Oryza sativa.* Trypsin was used to hydrolyze 10mg of each sample using standard conditions. Two peptides were identified and purified from these hydrolysates, namely the VWEKPWMDFK and PWMDFK peptides, as these exhibited the highest activities in the first screen. Again the hydrolysate and the two purified peptides were submitted to receptor-based screening. The hydrolysate itself inhibited CCK-8 binding to the CCK-B receptor by 21% at a 100 µM hydrolysate concentration, while the purified peptides, VWEKPWMDFK and PWMDFK, inhibited CCK-8 binding by 16%, and 14% at a 10 µM concentrations.

A final optimization of the hydrolysis by the combined consecutive action of trypsin and limited carboxypeptidase digestion was carried out with the goal of obtaining the peptides VWEKPWMDF and PWMDF, resulting from the removal of the C-terminal lysine residue. The hydrolysate enriched in these peptides exhibited an increased inhibition of the binding of the ligand CCK-8 to the CCK-B receptor that reached 38% at a 10 µM concentration of the active peptides, in comparison to the 21 % at the 10 times higher 100 µM concentration without trypsin treatment.

Trypsin digests were carried out by adding 96 mg urea to 200 µl oryzacystatin protein (2mg) and incubating at room temperature until the urea dissolves (final concentration: ca. 4 M urea). To the urea solution 900µl of 100mM ammonium bicarbonate in 1mM CaCl₂ is added followed by 120µl of acetonitrile. 40µg of trypsin (40µg Promega sequencing grade trypsin in Promega resuspension buffer) was added and the solution incubated overnight at 37 °C. A second aliquot of 10µg trypsin was added followed by incubation for 3 h at 37°C.

50% of the digestion solution of oryzacystatin is stored at -20°C for desalting by solid phase extraction using C18 reverse phase material and 50% of the digestion solution of oryzacystatin is subjected to carboxypeptiodase digestion.

Carboxypeptidase Y digestion was carried out on 720µl (ca. 1mg) of the tryptic digestion solution of oryzacystatin to which 1080µl of 200 mM acetate buffer, pH 5 in 10% acetonitrile was added. 100µg carboxypeptidase Y (Sigma Aldrich, carboxypeptidase Y solution was prepared three days before. It is good for one week according to Sigma Aldrich) is added and the solution incubated for 30 min at room temperature followed by the addition of 910 µl of 1% formic acid

A small fraction of each sample was characterized by nano LC-ESI-MSMS.

Both samples were desalted using 500µl C18 solid extraction columns with 0.1 % formic acid as washing solution and 80% acetonitrile/0.1% formic acid as elution solution.

## Claims

1. A process for developing a method for generating functional biomolecules consisting of a peptide defined by the amino acid sequence of SEQ-ID No. 8 or 9 from precursors comprising:
a. identifying a precursor food source that contains a precursor molecule comprising said functional biomolecule,
b. identifying an agent that can be used to release from the precursor said functional biomolecule.

2. The process for developing a method for generating functional biomolecules from precursors of Claim 1, wherein the functional biomolecule is a bioactive molecule.

3. The process for developing a method for generating functional biomolecules from precursors of Claim 1, wherein the precursor food source is a food stuff.

4. The process for developing a method for generating functional biomolecules from precursors of Claim 1, wherein the precursor food source is an enriched protein source.

5. The process for developing a method for generating functional biomolecules from precursors of Claim 1, wherein the agent is a protease.

6. A method for preparing a functional food enriched in a functional biomolecule comprising:
a. identifying a functional biomolecule consisting of a peptide defined by the amino acid sequence of SEQ-ID No. 8 or 9 to be generated,
b. obtaining an agent that can be used to release from the precursor molecule said functional biomolecule, and
c. treating the precursor food source with the processing agent to release from the precursor molecule the functional biomolecule.

7. The method of Claim 6, wherein the functional biomolecule is a bioactive molecule.

8. The method of Claim 6, wherein the precursor food source is a food grade protein.

9. The method of Claim 6, wherein the agent is a protease.

10. A method for preparing a functional molecule consisting of a peptide defined by the amino acid sequence of SEO-ID No. 8 or 9 comprising:
a identifying a precursor food source that contains a precursor molecule comprising said functional biomolecule,
b. identifying an agent that can be used to release from the precursor molecule said functional biomolecule, and
c. treating the precursor food source with the processing agent to release from the precursor molecule a molecule that contains the attributes necessary for function.

11. The method of claim 10 further comprising purifying the functional biomolecule from the processed precursor food source.

12. The method of claim 10 further comprising concentrating the functional biomolecule in the processed precursor food source.

13. The method of claim 10 further comprising preparing an extract of the processed precursor food source that contains an elevated concentration of the biomolecule.

14. The method of Claim 10 wherein the processing agent releases the biomolecule from the precursor molecule.

15. A food product comprising a digested precursor molecule and a biomolecule consisting of a peptide defined by the amino acid sequence of SEQ-ID No. 8 or 9.

## Patentansprüche

1. Prozess zur Entwicklung eines Verfahrens zum Erzeugen funktioneller Biomoleküle, die aus einem Peptid bestehen, das durch die Aminosäuresequenz der SEQ-ID Nr. 8 oder 9 definiert ist, aus Präkursoren, aufweisend:
a. Identifizieren einer Präkursor-Lebensmittelquelle, die ein Präkursormolekül enthält, das das funktionelle Biomolekül aufweist,
b. Identifizieren eines Agens, das verwendet werden kann, um das funktionelle Bio-molekül aus dem Präkursor freizusetzen.

2. Prozess zur Entwicklung eines Verfahrens zum Erzeugen von funktionellen Biomolekülen aus Präkursoren nach Anspruch 1, wobei das funktionelle Biomolekül ein bioaktives Molekül ist.

3. Prozess zur Entwicklung eines Verfahrens zum Erzeugen funktioneller Biomoleküle aus Präkursoren nach Anspruch 1, wobei die Präkursor-Lebensmittelquelle ein Nahrungsmittel ist.

4. Prozess zur Entwicklung eines Verfahrens zum Erzeugen funktioneller Biomoleküle aus Präkursoren nach Anspruch 1, wobei die Präkursor-Lebensmittelquelle eine angereicherte Proteinquelle ist.

5. Prozess zur Entwicklung eines Verfahrens zum Erzeugen funktioneller Biomoleküle aus Präkursoren nach Anspruch 1, wobei das Agens eine Protease ist.

6. Verfahren zur Herstellung eines funktionellen Lebensmittels, das mit einem funktionellen Biomolekül angereichert ist, aufweisend:
a. Identifizieren eines zu erzeugenden funktionellen Biomoleküls, das aus einem Peptid besteht, das durch die Aminosäuresequenz der SEQ-ID Nr. 8 oder 9 definiert ist,
b. Erhalten eines Agens, das verwendet werden kann, um das funktionelle Biomolekül aus dem Präkursormolekül freizusetzen, und
c. Behandeln der Präkursor-Lebensmittelquelle mit dem Verarbeitungsagens, um das funktionelle Biomolekül aus dem Präkursormolekül freizusetzen.

7. Verfahren nach Anspruch 6, wobei das funktionelle Biomolekül ein bioaktives Molekül ist.

8. Verfahren nach Anspruch 6, wobei die Präkursor-Lebensmittelquelle ein Protein mit Lebensmittelgüte ist.

9. Verfahren nach Anspruch 6, wobei das Agens eine Protease ist.

10. Verfahren zur Herstellung eines funktionellen Moleküls, das aus einem Peptid besteht, das durch die Aminosäuresequenz der SEQ-ID Nr. 8 oder 9 definiert ist, aufweisend:
a. Identifizieren einer Präkursor-Lebensmittelquelle, die ein Präkursormolekül enthält, das das funktionelle Biomolekül aufweist,
b. Identifizieren eines Agens, das verwendet werden kann, um das funktionelle Biomolekül aus dem Präkursormolekül freizusetzen und
c. Behandeln der Präkursor-Lebensmittelquelle mit dem Verarbeitungsagens, um ein Molekül aus dem Präkursormolekül freizugeben, das die zur Funktion notwendigen Eigenschaften enthält.

11. Verfahren nach Anspruch 10, das ferner das Aufbereiten des funktionellen Biomoleküls aus der verarbeiteten Präkursor-Lebensmittelquelle aufweist.

12. Verfahren nach Anspruch 10, das ferner das Aufkonzentrieren des funktionellen Biomoleküls in der verarbeiteten Präkursor-Lebensmittelquelle aufweist.

13. Verfahren nach Anspruch 10, das ferner das Herstellen eines Extrakts der verarbeiteten Präkursor-Lebensmittelquelle, die eine erhöhte Konzentration des Biomoleküls enthält, aufweist.

14. Verfahren nach Anspruch 10, wobei das Verarbeitungsagens das Biomolekül aus dem Präkursormolekül freisetzt.

15. Ein Nahrungsmittelprodukt, das ein aufgeschlossenes Präkursormolekül und Biomolekül aufweist, das aus einem Peptid besteht, das durch die Aminosäuresequenz der SEQ-ID Nr. 8 oder 9 definiert ist.

## Revendications

1. Procédé pour développer un procédé pour générer des biomolécules fonctionnelles, qui consistent en un peptide défini par la séquence d'acides aminés de SEQ-ID No. 8 ou 9, à partir de précurseurs et comprenant les étapes comprenant :
a. identifier une source alimentaire précurseur qui contient une molécule précurseur comprenant ladite biomolécule fonctionnelle,
b. identifier un agent qui peut être utilisé pour libérer du précurseur ladite biomolécule fonctionnelle.

2. Procédé pour développer un procédé pour générer des biomolécules fonctionnelles à partir de précurseurs selon la revendication 1,
dans lequel la biomolécule fonctionnelle est une molécule bioactive.

3. Procédé pour développer un procédé pour générer des biomolécules fonctionnelles à partir de précurseurs selon la revendication 1,
dans lequel la source alimentaire précurseur est une substance alimentaire.

4. Procédé pour développer un procédé pour générer des biomolécules fonctionnelles à partir de précurseurs selon la revendication 1,
dans lequel la source alimentaire précurseur est une source de protéine enrichie.

5. Procédé pour développer un procédé pour générer des biomolécules fonctionnelles à partir de précurseurs selon la revendication 1,
dans lequel l'agent est une protéase.

6. Procédé pour préparer un aliment fonctionnel enrichi dans une biomolécule fonctionnelle comprenant les étapes consistant à :
a. identifier une biomolécule fonctionnelle, qui consiste en un peptide défini par la séquence d'acides aminés de SEQ-ID No. 8 ou 9, à générer,
b. obtenir un agent qui peut être utilisé pour libérer de la molécule précurseur ladite biomolécule fonctionnelle, et
c. traiter la source alimentaire précurseur avec l'agent de traitement pour libérer de la molécule précurseur la biomolécule fonctionnelle.

7. Procédé selon la revendication 6,
dans lequel la biomolécule fonctionnelle est une molécule bioactive.

8. Procédé selon la revendication 6,
dans lequel la source alimentaire précurseur est une protéine alimentaire.

9. Procédé selon la revendication 6,
dans lequel l'agent est une protéase.

10. Procédé pour préparer une molécule fonctionnelle, qui consiste en un peptide défini par la séquence d'acides aminés de SEQ-ID No. 8 ou 9, comprenant les étapes consistant à :
a. identifier une source alimentaire précurseur qui contient une molécule précurseur comprenant ladite biomolécule fonctionnelle,
b. identifier un agent qui peut être utilisé pour libérer de la molécule précurseur ladite biomolécule fonctionnelle, et
c. traiter la source alimentaire précurseur avec l'agent de traitement pour libérer de la molécule précurseur une molécule qui contient les attributs nécessaires pour la fonction.

11. Procédé selon la revendication 10,
comprenant en outre l'étape consistant à purifier la biomolécule fonctionnelle de la source alimentaire précurseur traitée.

12. Procédé selon la revendication 10,
comprenant en outre la concentration de la biomolécule fonctionnelle dans la source alimentaire précurseur traitée.

13. Procédé selon la revendication 10,
comprenant en outre la préparation d'un extrait de la source alimentaire précurseur traitée qui contient une concentration élevée de la biomolécule.

14. Procédé selon la revendication 10,
dans lequel l'agent de traitement libère la biomolécule de la molécule précurseur.

15. Un produit alimentaire comprenant une molécule précurseur digérée et une biomolécule consistant en un peptide défini par la séquence d'acides aminés de SEQ-ID No. 8 ou 9.
